# FASCICULE DE BREVET EUROPEEN

(11) **EP 1 578 268 B1**
(45) Date de publication et mention de la délivrance du brevet: **13.02.2008**
(21) Numéro de dépôt: 03813160.3
(22) Date de dépôt: 10.12.2003
(51) Int. Cl.: A61B 5/117, G09F 3/00

(54) **PROCEDE D'IDENTIFICATION ET D'AUTHENTIFICATION SANS LECTEUR SPECIFIQUE D'UN IDENTIFICATEUR**
VERFAHREN ZUR IDENTIFIZIERUNG UND AUTHENTIFIZIERUNG OHNE SPEZIFISCHES LESE- UND IDENTIFIKATIONSGERÄT
METHOD FOR IDENTIFYING AND AUTHENTICATING WITHOUT SPECIFIC READER AN IDENTIFIER

(30) Priorité: 13.12.2002 FR 0215783
(43) Date de publication de la demande: 28.09.2005
(73) Titulaire: Novatec, 82000 Montauban (FR)
(72) Inventeur: BOURRIERES, Francis, F-82000 Montauban (FR); KAISER, Clément, F-82000 Montauban (FR)
(86) Numéro de dépôt international: PCT/FR2003/003668
(87) Numéro de publication internationale: WO 2004/054444

(56) Documents cités:
- EP-A- 0 681 252
- WO-A-98/02083
- US-A- 3 805 238
- US-A- 4 614 366
- US-A- 5 839 215

## Description

### DOMAINE D'APPLICATION

La présente invention vise à proposer un moyen pour identifier et authentifier un identificateur rattaché à des objets ou à des êtres vivants sans nécessiter l'utilisation d'un lecteur spécifique. Elle trouve son application dans la lutte contre la contrefaçon, la traçabilité ou la sécurité.

### ETAT DE L'ART

A ce jour, plusieurs moyens existent pour identifier ou authentifier les objets ou les êtres vivants. Un moyen répandu consiste à apposer un hologramme sur l'objet à contrôler, ainsi lors d'une transaction, la présence de l'hologramme est censée garantir l'authenticité du produit. Or avec les moyens de reproduction et d'impression actuels, il est aisé de copier et de reproduire un hologramme et par conséquent d'apposer de faux hologrammes sur des produits contrefaits. De plus, l'acquéreur n'a à priori aucune information sur le fait qu'un hologramme doit être présent sur l'objet et encore moins sur l'aspect que doit avoir cet hologramme. Par conséquent, si l'article ne présente pas d'hologramme ou pire encore, si ce dernier représente vaguement la marque de l'objet qu'il est censé authentifier, l'acquéreur sera berné.

Une autre technique, consiste à associer une puce électronique ou une étiquette radio-fréquence appelée RFID à l'objet à authentifier. Cette solution repose sur la complexité et l'investissement que nécessite la réalisation de ce type d'identificateur ainsi que sur des secrets partagés comme des algorithmes de cryptage. Cette technique présente deux inconvénients majeurs qui limitent considérablement sa généralisation qui sont d'une part le coût important des identificateurs qui est typiquement supérieur à un euro, et d'autre part, la nécessité d'accès à un lecteur spécifique pour lire et interpréter l'identificateur.

La demande de brevet PCT/FR01.00322 du même inventeur décrit un identificateur non reproductible basé sur une distribution aléatoire d'hétérogénéités dans une matière transparente. L'identification et l'authentification de l'identificateur est faite à l'aide d'un lecteur spécifique qui permet de soumettre l'identificateur à deux éclairages différents et de comparer la signature de l'identificateur présenté avec celle enregistrée dans une base de données. La nécessité de faire appel à un lecteur spécifique est ici encore un frein majeur à l'extension de cette technologie en particulier pour les applications grand public.

Le document US 3,805,238 concerne un procédé d'identification automatique ou visuel d'une personne physique à partir d'un ou de plusieurs traits caractéristiques particuliers de la personne physique qui auront préalablement été enregistrés soit dans une base de données soit directement sur un élément tel qu'une carte d'identité. Le procédé décrit dans ce document, s'applique donc à une personne qui a la particularité de présenter des caractéristiques physiques remarquables qui peuvent être directement utilisées pour identifier ladite personne. Dans la présente invention comme cela sera vu plus loin, la demanderesse a cherché à proposer un mode d'identification indirect en proposant un identificateur qui est rattaché à un objet ou à un être vivant que l'on souhaite marquer et non à identifier ou à authentifier l'objet ou l'être vivant lui-même. Par conséquent l'objet ou l'être vivant à marquer n'a pas nécessairement besoin de présenter de caractéristique remarquable. Au contraire, dans le cas de la lutte contre la contrefaçon le présent procédé, objet de l'invention permet de marquer des objets identiques entre eux. A cet effet, l'inventeur suggère d'utiliser des identificateurs tridimensionnels présentant une distribution aléatoire d'hétérogénéités rendant ceux-ci toujours uniques et impossibles ou très difficiles à reproduire.

Le document US 5,839,215 concerne une étiquette tactile en vue de donner des informations sur le produit sur lequel elle est apposée. Ce type d'étiquette ne poursuit pas un but d'authentification dans la mesure où elles sont faciles à produire et à reproduire à l'identique. En aucun cas ces étiquettes sont basées sur une distribution aléatoire d'hétérogénéités. Ces étiquettes ne nécessitent pas non plus la comparaison avec une image stockée dans une base de données. En fait ce type d'étiquette poursuit uniquement un but de traçabilité mais ne constitue en aucune manière un moyen de lutte contre la contrefaçon et encore moins un aspect sécuritaire. '

Le document WO 98/02083 concerne un dispositif et une méthode de test de capacités ou d'aptitudes de manière automatique. Ce brevet ne concerne en aucune façon un procédé d'identification et d'authentification sans lecteur spécifique d'un identificateur rattaché à un objet ou à un être vivant.

### DESCRIPTION DE L'INVENTION :

La présente invention vise à proposer un procédé humain pour identifier et authentifier des identificateurs rattachés à des objets ou à des êtres vivants sans les inconvénients de l'art antérieur cité. Elle vise plus particulièrement à offrir un moyen d'identification et d'authentification sans avoir besoin d'un lecteur spécifique. L'innovation consiste à utiliser les possibilités offertes par les moyens modernes de communications tels que Internet ou les dernières générations de téléphones mobiles dotés d'options multimédia. Pour la mise en oeuvre de l'invention, on rattache un identificateur difficile ou impossible à reproduire, à l'objet ou à l'être vivant que l'on souhaite marquer. De façon générale l'identificateur fait partie de la famille des identificateurs tridimensionnels, ceux-ci peuvent présenter soit une empreinte tridimensionnelle de surface en relief ou en creux, soit un arrangement aléatoire hétérogène quelconque à l'intérieur du volume. L'invention se caractérise essentiellement en ce que l'authentification est faite par vérification sensorielle humaine de la particularité qui rend l'identificateur difficile ou impossible à reproduire et que son identification ou sa lecture est faite par comparaison visuelle entre une image bidimensionnelle de l'identificateur stockée dans une base de données accessible par un réseau et l'identificateur lui-même. Ainsi, cette invention permet de reconnaître et d'identifier de façon indirecte des objets identiques, ce qui est particulièrement nouveau et inventif.

A titre d'exemple non limitatif, il peut être judicieux d'utiliser des identificateurs volumiques tridimensionnels constitués par une distribution aléatoire d'hétérogénéités dans un volume constitué d'une matière transparente ou translucide. Dans ce cas et selon une autre caractéristique de l'invention, l'authentification est faite par vérification visuelle stéréoscopique de l'aspect volumique tridimensionnel de l'identificateur et l'identification est faite par comparaison visuelle d'une image de représentation bidimensionnelle de l'identificateur, ladite image étant stockée dans une base de données accessible par un réseau, et de l'identificateur lui-même. Il s'agit ici d'utiliser avantageusement les capacités de l'oeil humain pour comparer de façon relative un objet et son image. Si la matière contenant la distribution aléatoire d'hétérogénéités est seulement visible sous éclairage infrarouge, il sera utilisé un capteur adapté placé entre les yeux et l'identificateur, pour apprécier visuellement s'il s'agit bien d'une distribution volumique.

Selon une autre caractéristique de l'invention, afin de faciliter l'identification visuelle, on peut reproduire une image semblable à l'image bidimensionnelle de l'identificateur qui est physiquement associée à l'identificateur tridimensionnel. Dans ce cas, l'identification est faite par une première comparaison visuelle entre l'identificateur volumique et l'image semblable associée, puis, par une deuxième comparaison visuelle entre l'image bidimensionnelle stockée dans la base de données et l'image semblable. Il va de soit que l'image semblable qui est associée à l'identificateur est représentée à une échelle appropriée afin de faciliter la comparaison. Pour faciliter la comparaison, il peut être judicieux de compléter l'image semblable et/ou l'image bidimensionnelle et/ou l'identificateur avec un marquage particulier. A titre d'exemple, ce marquage pourrait être un quadrillage et/ou une mire et/ou des axes orthogonaux.

De façon avantageuse, la présente invention peut être mise en oeuvre par le biais d'étiquettes ou de cartes d'accès à un droit ou à un service.

Selon une autre caractéristique de l'invention, le réseau qui permet d'accéder à la base de données est un réseau de télécommunications.

A titre d'exemple non limitatif, l'accès peut se faire par le biais du réseau internet. Selon une autre possibilité, il est envisageable d'accéder au contenu de la base de données et en particulier à l'image bidimensionnelle qui y est stockée à l'aide d'un téléphone portable présentant des fonctionnalités multimédias.

De façon avantageuse et selon une autre caractéristique de l'invention, l'identificateur peut être rattaché de manière inviolable à l'objet à identifier ou à authentifier.

D'autres caractéristiques de l'invention apparaîtront à la lecture des figures suivantes données à titre d'exemples non limitatifs.

La figure 1 représente un mode de vérification d'un identificateur dont la particularité est de présenter des hétérogénéités internes.

La figure 2 représente un mode de vérification d'un identificateur dont la particularité est de présenter des aspérités de surface.

La figure 3 représente un mode de vérification d'un identificateur dont la particularité est de présenter des cavités.

La figure 4 représente un mode de vérification d'un identificateur mixte dont la particularité est de présenter des aspérités de surface et des cavités.

La figure 5 représente un mode de mise en oeuvre de l'invention.

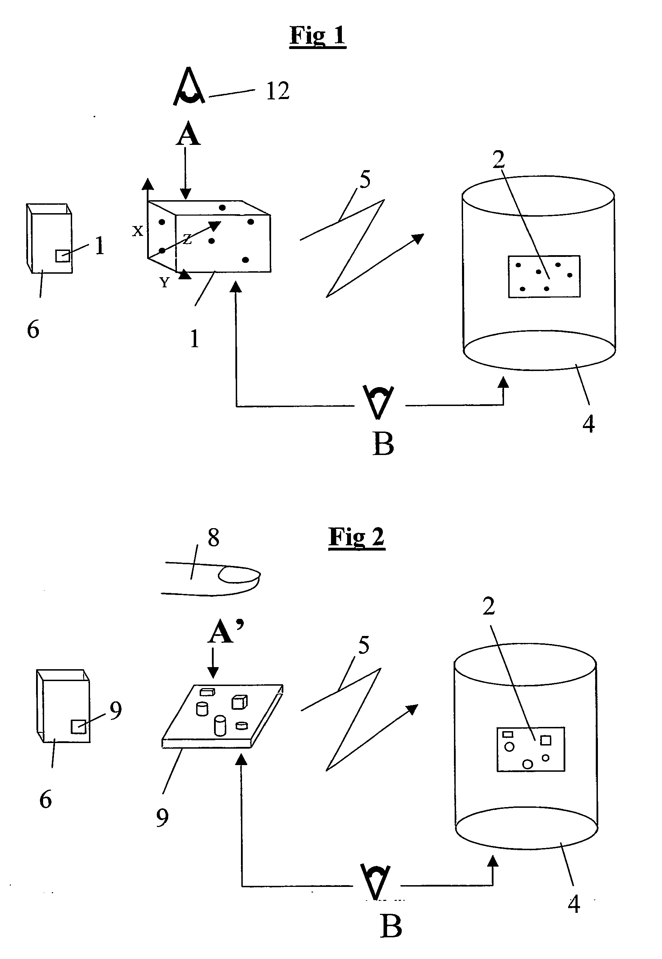

La figure 1 représente un procédé d'identification et d'authentification sans lecteur spécifique d'un identificateur volumique (1) dont la particularité est de présenter des hétérogénéités internes se présentant sous forme de bulles et/ou de particules solides réparties de façon aléatoire à l'intérieur d'une matière transparente. Pour vérifier l'authenticité de la distribution volumique des hétérogénéités, il est fait usage de la vision stéréoscopique (A) de l'oeil humain (12) et pour procéder à l'identification ou à la lecture, une comparaison visuelle (B) est effectuée entre l'image de représentation bidimensionnelle (2) de l'identificateur (1) stockée dans une base de données (4) accessible par un réseau (5) et l'identificateur (1) lui-même.

La figure 2 représente un procédé d'identification et d'authentification sans lecteur spécifique d'un identificateur volumique (9) dont la particularité est de présenter des aspérités de surface placées en relief. Pour vérifier l'authenticité de cette particularité, il est fait usage de la sensation tactile (A') d'un doigt (8) et pour procéder à l'identification ou à la lecture, une comparaison visuelle (B) est effectuée entre l'image de représentation bidimensionnelle (2) de l'identificateur (1) stockée dans une base de données (4) accessible par un réseau (5) et l'identificateur (9) lui-même.

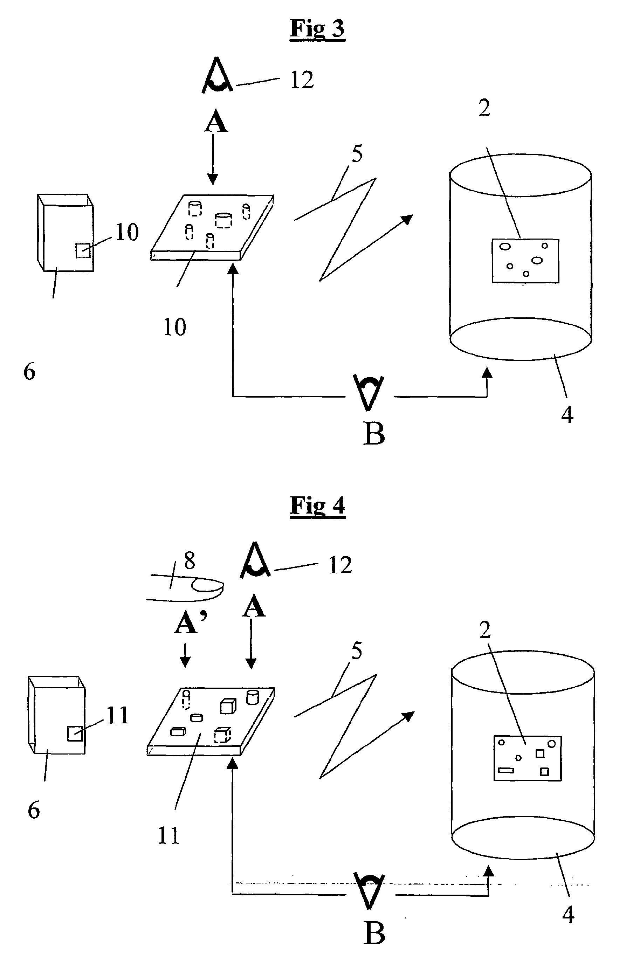

La figure 3 représente un procédé d'identification et d'authentification sans lecteur spécifique d'un identificateur volumique (10) dont la particularité est de présenter des cavités ménagées à partir de la surface dudit identificateur (10). Pour vérifier l'authenticité de cette particularité, il est fait usage de la vision stéréoscopique (A) de l'oeil humain (12) et pour procéder à l'identification ou à la lecture, une comparaison visuelle (B) est effectuée entre l'image de représentation bidimensionnelle (2) de l'identificateur (1) stockée dans une base de données (4) accessible par un réseau (5) et l'identificateur (10) lui-même.

La figure 4 représente un procédé d'identification et d'authentification sans lecteur spécifique d'un identificateur volumique mixte (11) dont la particularité est de présenter des aspérités de surface placées en relief et des cavités ménagées à partir de la surface dudit identificateur (11). Pour vérifier l'authenticité de ces particularités, il est fait usage de la sensation tactile (A') d'un doigt (8) et de la vision stéréoscopique (A) de l'oeil humain (12) et pour procéder à l'identification ou à la lecture, une comparaison visuelle (B) est effectuée entre l'image de représentation bidimensionnelle (2) de l'identificateur (1) stockée dans une base de données (4) accessible par un réseau (5) et l'identificateur (11) lui-même.

Selon un premier mode de mise en oeuvre de l'invention, l'identificateur volumique (1) (9) (10) (11) est relié à un objet ou à un être vivant (6), puis une image bidimensionnelle (2) de l'identificateur est enregistrée dans la base de données (4). La base de données (4) peut également comporter des informations complémentaires comme par exemple une description, un âge, une date de fabrication, une photographie, ... de l'objet ou de l'être vivant auquel l'identificateur est affecté. Lorsque ultérieurement, on cherche à faire une identification et une authentification de l'objet ou de l'être vivant (6) auquel l'identificateur (1) (9) (10) (11) a été affecté, on commence par faire une vérification sensorielle, qui peut être visuelle (A), ou tactile (A') qui permet dans le cas présent d'authentifier la ou les particularités de l'identificateur (1) (9) (10) (11). Ceci permet de garantir que l'on a à faire à un identificateur et non à une reproduction. Puis on vérifie l'identité de l'identificateur ou sa lecture en faisant une comparaison visuelle (B) entre l'identificateur (1) (9) (10) (11) et son image bidimensionnelle (2) stockée dans la base données, cette dernière étant accessible à travers un réseau (5). Il va de soit que si on le souhaite, on peut d'abord procéder à l'identification puis à l'authentification sans sortir du champ de la présente invention. Evidemment pour accéder directement à l'information autorisée et correspondante à l'identificateur (1) (9) (10) (11) dans la base données (4) il est judicieux d'adjoindre un numéro d'appel et éventuellement un mot de passe à l'identificateur pour sécuriser l'accès à la base de données. Ainsi, au numéro d'appel correspond une adresse dans la base de données.

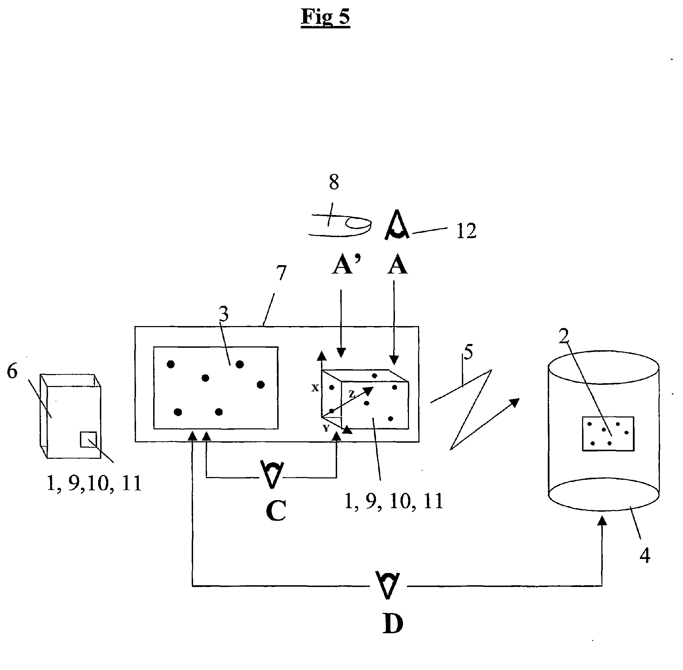

La figure 5 représente un autre mode de mise en oeuvre de la présente invention. Dans ce cas, l'identificateur (1) (9) (10) (11) est associé à une étiquette ou une carte (7), elle-même rattachée virtuellement ou de manière physique à l'objet ou à l'être vivant (6) à identifier. Dans le cas présent et de manière à faciliter les opérations d'identification, une image semblable (3) représentant les particularités de l'identificateur est imprimée sur l'étiquette ou la carte à une échelle appropriée. Ainsi pour procéder à une première identification, on fait une première comparaison visuelle (C) entre l'identificateur (1) (9) (10) (11) et l'image semblable (3) puis une deuxième comparaison visuelle (D) entre l'image semblable (3) et l'image bidimensionnelle (2) contenue dans la base de données. Pour les identifications ultérieures, il suffira de procéder à la comparaison visuelle (D) entre l'image semblable (3) et l'image bidimensionnelle (2).

De façon avantageuse, l'image semblable (3) et l'image bidimensionnelle (2) sont à des échelles identiques de manière à faciliter leur comparaison.

Selon une autre caractéristique de l'invention, afin de rendre l'identificateur inviolable, il peut être judicieux de le coller sur l'objet à identifier, de sorte que si l'on cherche à le décoller on le détériore de façon irréversible.

La présente invention est utilisable pour un grand nombre d'applications car elle permet à tout utilisateur d'accéder de façon très simple et sans investissement à la vérification de l'authenticité d'un produit et à ses caractéristiques essentielles en utilisant simplement les capacités sensorielles de l'être humain qui permettent de faire le lien entre une base de données et un identificateur, difficile ou impossible à reproduire, affecté à un objet. Il est par exemple possible de vérifier les identités biométriques d'êtres humains (empreintes digitales, iris de l'oeil) sans avoir à utiliser des appareils d'interprétation et de lecture coûteux.

## Revendications

1. Procédé d'identification et d'authentification indirect et sans lecteur d'un objet à l'aide d'un identificateur tridimensionnel rattaché audit objet, l'identificateur présentant des hétérogénéités réparties de façon aléatoire à l'intérieur d'une matière transparente rendant celui-ci difficile ou impossible à reproduire, faisant usage de la vision stéréoscopique (A) de l'oeil humain (12) pour vérifier l'aspect tridimensionnel garantissant l'authenticité dudit identificateur (1), et que l'identification ou la lecture étant faite par comparaison visuelle (B) d'une image bidimensionnelle (2) de l'identificateur (1) stockée dans une base de données (4) accessible par un réseau (5), et l'identificateur lui-même (1).

2. Procédé d'identification et d'authentification indirect et sans lecteur d'un objet à l'aide d'un identificateur tridimensionnel rattaché audit objet, l'identificateur présentant des hétérogénéités réparties de façon aléatoire à l'intérieur d'une matière transparente rendant celui-ci difficile ou impossible à reproduire, selon la revendication 1, **caractérisé en ce que** pour faciliter l'identification ou la lecture visuelles, on reproduise, à une échelle appropriée, une image semblable (3) à l'image bidimensionnelle (2) de l'identificateur (1) (9) (10) (11), ladite image (2) étant stockée dans la base de données (4), et que l'image semblable (3) soit physiquement associée à l'identificateur tridimensionnel (1). (9) (10) (11) afin d'effectuer une première comparaison visuelle (C) entre ledit identificateur volumique (1) (9) (10) (11) et l'image semblable (3) et une deuxième comparaison visuelle (D) entre l'image (2) dans sa version stockée dans la base de données (4) et sa représentation semblable (3) associée à l'identificateur volumique (1) (9) (10) (11).

3. Procédé d'identification et d'authentification indirect et sans lecteur d'un objet à l'aide d'un identificateur tridimensionnel rattaché audit objet, l'identificateur présentant des hétérogénéités réparties de façon aléatoire à l'intérieur d'une matière transparente rendant celui-ci difficile ou impossible à reproduire selon la revendication 1, **caractérisé en ce qu'**il est fait usage d'un numéro d'appel et éventuellement d'un mot de passe associé à l'identificateur afin de faciliter et sécuriser l'accès à la base de données (4).

4. Procédé d'identification et d'authentification indirect et sans lecteur d'un objet à l'aide d'un identificateur tridimensionnel rattaché audit objet, l'identificateur présentant des hétérogénéités réparties de façon aléatoire à l'intérieur d'une matière transparente rendant celui-ci difficile ou impossible à reproduire selon la revendication 1, **caractérisé en ce que** le réseau (5) est un réseau de télécommunication.

5. Procédé d'identification et d'authentification indirect et sans lecteur d'un objet à l'aide d'un identificateur tridimensionnel rattaché audit objet, l'identificateur présentant des hétérogénéités réparties de façon aléatoire à l'intérieur d'une matière transparente rendant celui-ci difficile ou impossible à reproduire selon les revendications 1 à 4 prises ensemble ou séparément, **caractérisé en ce que** l'identificateur (1) (9) (10) (11) et/ou l'image bidimensionnelle (2) et/ou l'image semblable (3) présentent un marquage ou repérage particulier afin de faciliter la ou les comparaisons visuelles.

## Claims

1. Identification and authentification process, indirect and without reader, of an object with the assistance of a three-dimensional identifier attached to the object, the identifier presenting heterogenities distributed in a random manner within a transparent material rendering the latter difficult or impossible to reproduce using the stereoscopic vision (A) of the human eye (12) to verify the three-dimensional appearance guaranteeing the authenticity of the aforementioned identifier (1) and that the identification or reading being made by visual comparison (B) of a two-dimensional image (2) of the identifier (1) stored in a database (4) accessible by a network (5), and the identifier himself (1).

2. Identification and authentification process, indirect and without reader, of an object with the assistance of a three-dimensional identifier attached to the object, the identifier presenting heterogenities distributed in a random manner within a transparent material rendering the latter difficult or impossible to reproduce, pursuant to Claim 1, **characterized by** the fact that to facilitate the identification or visual reading, one reproduces at an appropriate scale a similar image (3) to the two-dimensional image (2) of the identifier (1) (9) (10) (11), the afore-mentioned image (2) being stored in the database (4), and that the similar image (3) is physically associated with the three-dimensional identifier (1) (9) (10) (11) to carry out a first visual comparison (C) between the volumic identifier (1) (9) (10) (11) and the similar image (3) and a second visual comparison (D) between the image (2) in its version stored in the database (4) and its similar representation (3) associated with the volumic identifier (1) (9) (10) (11).

3. Identification and authentification process, indirect and without reader, of an object with the assistance of a three-dimensional identifier attached to the object, the identifier presenting heterogenities distributed in a random manner within a transparent material rendering the latter difficult or impossible to reproduce, pursuant to Claim 1, **characterized by** the fact that it uses a call number and possibly a password associated with the identifier in order to facilitate and secure access to the database (4).

4. Identification and authentification process, indirect and without reader, of an object with the assistance of a three-dimensional identifier attached to the object, the identifier presenting heterogenities distributed in a random manner within a transparent material rendering the latter difficult or impossible to reproduce, pursuant to Claim 1, **characterized by** the fact that the network (5) is a telecommunications network.

5. Identification and authentification process, indirect and without reader, of an object with the assistance of a three-dimensional identifier attached to the object, the identifier presenting heterogenities distributed in a random manner within a transparent material rendering the latter difficult or impossible to reproduce, pursuant to Claims 1 to 4 taken together or separately, **characterized by** the fact that the identifier (1) (9) (10) (11) and/or two-dimensional image (2) and/or similar image (3) present a particular marking or identification in order to facilitate the visual comparison(s).

## Patentansprüche

1. Verfahren zur indirekten Identifizierung und Authentifizierung ohne Lesegerät eines Objekts mit Hilfe einer an dem Objekt angeschlossenen dreidimensionalen Kennzeichnung, wobei die Kennzeichnung auf zufällige Weise im Inneren eines transparenten Materials verteilte Heterogenitäten aufweist, was diese schwierig oder unmöglich zu reproduzieren macht, wobei es die stereoskopische Sicht (A) des menschlichen Auges (12) gebraucht, um die dreidimensionale Erscheinungsform zu überprüfen, welche die Authentizität der Kennzeichnung (1) gewährleistet, und wobei die Identifizierung oder das Lesen durch einen visuellen Vergleich (B) eines zweidimensionalen Bildes (2) der Kennzeichnung (1), welches in einer über ein Netz (5) zugänglichen Datenbank (4) gespeichert ist, und der , Kennzeichnung selbst (1) erfolgen.

2. Verfahren zur indirekten Identifizierung und Authentifizierung ohne Lesegerät eines Objekts mit Hilfe einer an dem Objekt angeschlossenen Kennzeichnung, wobei die Kennzeichnung auf zufällige Weise im Inneren eines transparenten Materials verteilte Heterogenitäten aufweist, was diese schwierig oder unmöglich zu reproduzieren macht, nach Anspruch 1, **dadurch gekennzeichnet, dass** um die visuelle Identifizierung oder das visuelle Lesen zu erleichtern in einem geeigneten Maßstab ein zu dem zweidimensionalen Bild (2) der Kennzeichnung (1) (9) (10) (11) ähnliches Bild (3) wiedergegeben wird, wobei das Bild (2) in der Datenbank (4) gespeichert ist, und dass das ähnliche Bild (3) physikalisch mit der dreidimensionalen Kennzeichnung (1) (9) (10) (11) verknüpft ist, so dass ein erster visueller Vergleich (C) zwischen der Volumenkennzeichnung (1) (9) (10) (11) und dem ähnlichen Bild (3) und ein zweiter visueller Vergleich (D) zwischen dem Bild (2) in seiner in der Datenbank (4) gespeicherten Version und seiner mit der Volumenkennzeichnung (1) (9) (10) (11) verknüpften ähnlichen Darstellung (3) erfolgen.

3. Verfahren zur indirekten Identifizierung und Authentifizierung ohne Lesegerät eines Objekts mit Hilfe einer an dem Objekt angeschlossenen dreidimensionalen Kennzeichnung, wobei die Kennzeichnung auf zufällige Weise im Inneren eines transparenten Materials verteilte Heterogenitäten aufweist, was diese schwierig oder unmöglich zu reproduzieren macht, nach Anspruch 1, **dadurch gekennzeichnet, dass** es eine Rufnummer und unter Umständen ein Passwort gebraucht, welches mit der Kennzeichnung verknüpft ist, um den Zugriff auf die Datenbank (4) zu erleichtern und abzusichern.

4. Verfahren zur indirekten Identifizierung und Authentifizierung ohne Lesegerät eines Objektes mit Hilfe einer an dem Objekt angeschlossenen dreidimensionalen Kennzeichnung, wobei die Kennzeichnung auf zufällige Weise im Inneren eines transparenten Materials verteilte Heterogenitäten aufweist, was diese schwierig oder unmöglich zu reproduzieren macht, nach Anspruch 1, **dadurch gekennzeichnet, dass** das Netz (5) ein Telekommunikationsnetz ist.

5. Verfahren zur indirekten Identifizierung und Authentifizierung ohne Lesegerät eines Objektes mit Hilfe einer an dem Objekt angeschlossenen dreidimensionalen Kennzeichnung, wobei die Kennzeichnung auf zufällige Weise im Inneren eines transparenten Materials verteilte Heterogenitäten aufweist, was diese schwierig oder unmöglich zu reproduzieren macht, nach den Ansprüchen 1-4 zusammengenommen oder separat, **dadurch gekennzeichnet, dass** die Kennzeichnung (1) (9) (10) (11) und/oder das zweidimensionale Bild (2) und/oder das ähnliche Bild (3) eine besondere Markierung oder Bezeichnung aufweisen, um den visuellen Vergleich oder die visuellen Vergleiche zu erleichtern.
